Europäisches Patentamt

European Patent Office

Office européen des brevets

(19)

(11) Veröffentlichungsnummer: **0 236 894 B1**

(12) **EUROPÄISCHE PATENTSCHRIFT**

(45) Veröffentlichungstag der Patentschrift: **09.11.94**

(21) Anmeldenummer: **87102909.6**

(22) Anmeldetag: **02.03.87**

(51) Int. Cl.5: **C07H 7/06**, A23K 1/17,
C12P 19/12, //(C12P19/12,
C12R1:465)

(54) **Efomycin G, seine Herstellung und seine Verwendung als Leistungsförderer bei Tieren.**

(30) Priorität: **12.03.86 DE 3608175**

(43) Veröffentlichungstag der Anmeldung:
**16.09.87 Patentblatt 87/38**

(45) Bekanntmachung des Hinweises auf die
Patenterteilung:
**09.11.94 Patentblatt 94/45**

(84) Benannte Vertragsstaaten:
**AT BE CH DE ES FR GB GR IT LI NL SE**

(56) Entgegenhaltungen:
EP-A- 0 197 360
DE-A- 3 248 280

HELVETICA CHIMICA ACTA, Band 64, Nr. 41,
Fasc. 2, 1981, Seiten 407-424,Schweizerische
Chemische Gesellschaft; H. KAISER et al.:
"Stoffwechselproduktevon Mikroorganismen. Strukturaufklärung von Elaiophylin:
Spektroskopische Untersuchungen und Abbau"

(73) Patentinhaber: **BAYER AG**

**D-51368 Leverkusen (DE)**

(72) Erfinder: **Frobel, Klaus, Dr.**
**Birkenhöhe 5**
**D-5600 Wuppertal 1 (DE)**
Erfinder: **Müller, Hartwig, Dr.**
**Steinstrasse 15**
**D-5620 Velbert 15 (DE)**
Erfinder: **Bischoff, Erwin, Dr.**
**Pahlkestrasse 73**
**D-5600 Wuppertal 1 (DE)**
Erfinder: **Salcher, Olga, Dr.**
**Am Eckbusch 43/110**
**D-5600 Wuppertal 1 (DE)**
Erfinder: **De Jong, Anno, Dr.**
**Stockmannsmühle 46**
**D-5600 Wuppertal 1 (DE)**
Erfinder: **Berschauer, Friedrich, Dr.**
**Claudiusweg 9**
**D-5600 Wuppertal 1 (DE)**
Erfinder: **Scheer, Martin, Dr.**
**H.-Katernberg 7**
**D-5600 Wuppertal 1 (DE)**

EP 0 236 894 B1

**Beschreibung**

Die vorliegende Erfindung betrifft Efomycin G, seine Herstellung und seine Verwendung als Leistungsförderer bei Tieren.

Die EP-A-0 197 360 beschreibt die Efomycine A - D und F, enthält aber keinen Hinweis auf Efomycin G.

Die DE-A-3 248 280 beschreibt ein Antibiotikum mit Namen Salbomycin, welches strukturelle Ähnlichkeiten mit dem Efomycin G hat. Dieses Zitat enthält aber keinen Hinweis auf die Verwundbarkeit von Efomycin G als Leistungsförderer bei Tieren.

1. Es wurde das Efomycin G mit den im folgenden angegebenen chemischen und physikalischen Eigenschaften gefunden:

1. Die Summenformel $C_{53}H_{86}O_{18}$

2. Das Massenspektrum (Fast Atom Bombardment) Molmasse + $Na^+$: 1033

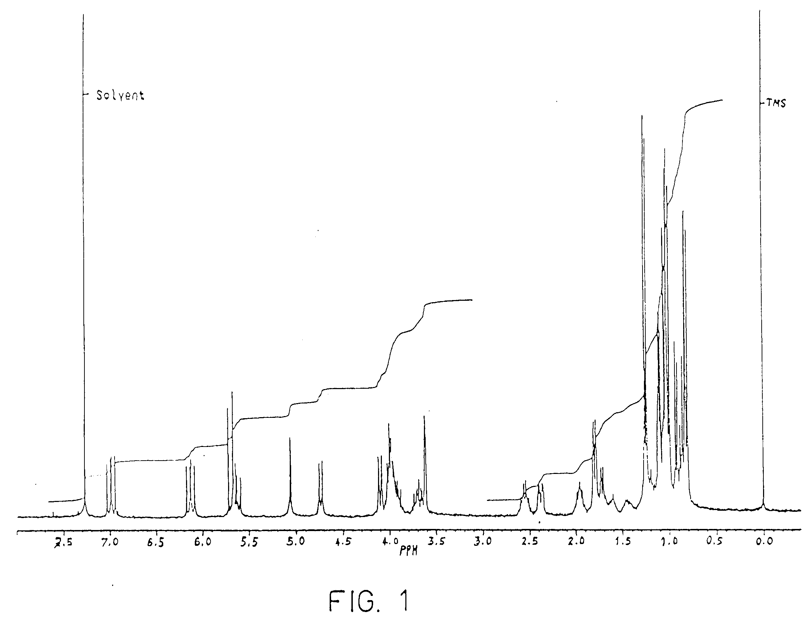

3. Das $^1$H-Kernresonanzspektrum, angegeben in Teilen pro Million, gemäß Fig. 1

Es wurde in einem AM 300 der Fa. Bruker bei MHz an einer Lösung von Efomycin G in deuteriertem Chloroform mit Tetramethylsilan als innerem Standard aufgenommen.

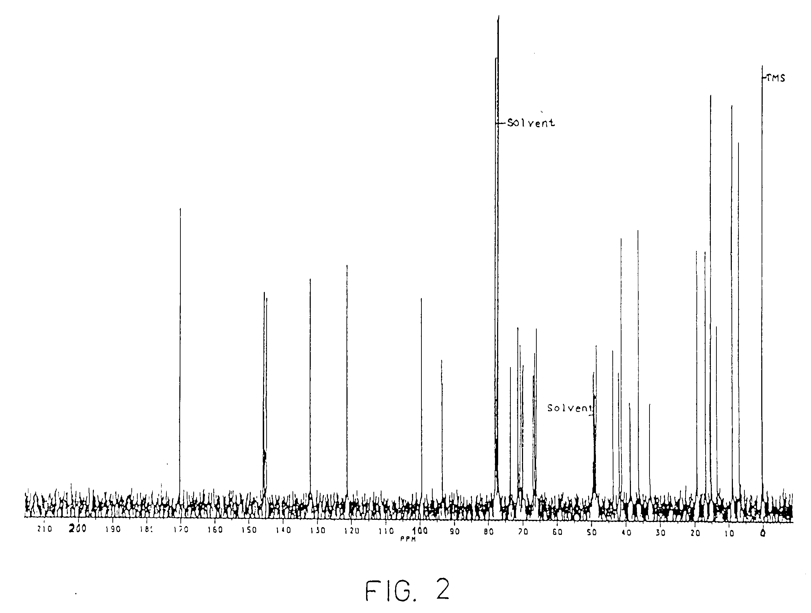

4. Das $^{13}$C-Kernresonanzspektrum, angegeben in Teilen pro Million, gemäß Fig. 2.

Es wurde in einem AM 300 der Fa. Bruker bei 75,48 MHz an einer Lösung von Efomycin G in deuteriertem Chloroform und deuteriertem Methanol mit Tetramethylsilan als innerem Standard aufgenommen.

Die chemischen Verschiebungen der $^{13}$C-NMR Signale von Efomycin G gemäß Abb. 2 betragen im einzelnen:

| | | | | |
|---|---|---|---|---|
| 170.0 | 77.9 | 65.9 | 32.9 | 7.0 |
| 145.4 | 73.4 | 48.5 | 19.4 | |
| 144.7 | 71.2 | 43.6 | 19.1 | |
| 131.9 | 70.6 | 41.9 | 16.8 | |
| 121.1 | 70.1 | 41.1 | 16.7 | |
| 99.6 | 69.9 | 38.8 | 15.1 | |
| 99.5 | 66.9 | 38.5 | 13.4 | |
| 93.6 | 66.5 | 36.2 | 9.0 | |
| 93.3 | 66.4 | 33.0 | 8.9 | |

(angegeben sind ppm-Werte relativ zu Tetramethylsilan bei 0 ppm)

5. Das UV-Absorptionsmaximum bei 251 - 254 nm in methanolischer Lösung.

6. Die Struktur gemäß der in Fig. 3 abgebildeten Strukturformel.

2. Es wurde gefunden, daß man das erfindungsgemäße Efomycin G erhält, wenn man geeignete Mikroorganismen der Familie der Streptomycetaceae in einem Nährmedium welches assimilierbare Kohlenstoff- und Stickstoffquellen sowie Mineralsalze enthält unter aeroben Bedingungen kultiviert, das entstandene Gemisch der Efomycine nach üblichen Methoden isoliert und trennt.

Bei Kenntnis der Eigenschaften des Efomycin G ist es mit Hilfe der üblichen chromatografischen, spektroskopischen und/oder biologischen Nachweisverfahren möglich, geeignete Mikroorganismenstämme auszumachen die Efomycin G produzieren.

Zur Durchführung des Verfahrens kann insbesondere der Streptomyces Stamm BS 1261 - sowie seine Mutanten und Varianten - Verwendung finden.

Dieser Stamm gehört zu der Familie Streptomycetaceae, der Gattung Streptomyces aus der grauen Serie der Streptomyceten (Cinereus-Gruppe).

Der Stamm BS 1261 wurde am 16.01.1985 bei der Deutschen Sammlung für Mikroorganismen (DSM) Grisebachstraße 8, 3400 Göttingen, Bundesrepublik Deutschland unter der Nummer DSM 3200 hinterlegt.

Taxonomische Beschreibung des Stamms BS 1261 (DSM 3200)

Die taxonomische Beschreibung des Stamms BS 1261 erfolgte nach Bergey's Manual of Determinative Bacteriology 8th, (1974) sowie International Journal of Systematic Bacteriology 16, 313-340 (1966) und The Prokaryotes 2,, 2028-2020 (1981).

## 1. Morphologie

Eine gute Sporulation war auf den ISP-Medien Nr. 2, 3, 4, 5, 7 bis 9 zu beobachten. Auf ISP-Medium Nr. 9 mit Ribose als C-Quelle sowie aus ISP-Medium Nr. 1 und 6 wurde überwiegend Substratmyzel gebildet.

Luftmyzel (ISP-Medium Nr. 3, 28°C, 7 Tage:

|  |  |
|---|---|
| Farbe: | grau (Cinereus-Typ) |
| Sporenketten: | Retinaculum-Apertum-Typ |
| Sporen: | Quadratisch bis rechteckig, 1,4-1,8 σm lang und 1,3-1,6 μm breit glatt (Elektronenmikroskopie). |

Substratmyzel:

|  |  |
|---|---|
| Farbe: | braun |

## 2. Angaben zur Physiologie

Das Temperaturoptimum liegt bei 28°C (auf ISP-Medium Nr. 2, 5 Tage). Der Stamm wächst nicht bei 4° und 45°C. Melanin wird nicht gebildet. Das Wachstum wird durch die Antibiotika Erythromycin (10 σg), Sulphafurazol (100 μg), Streptomycin (10 μg) und Novobiocin (5 μg) gehemmt (ISP-Medium Nr. 2, 28°C, 2 Tage).

Die Verwertung von C-Quellen wurde auf Basalagar (ISP-Medium Nr. 9) nach Int. Syst. Bact. 16, 313-340 (1966) überprüft. Für die Negativ-Kontrolle wurde das Wachstum auf Basalagar ohne C-Quelle verglichen. Dabei werden folgende Ergebnisse erhalten:

Tabelle

| Verwertung von C-Quellen durch Stamm BS 1261 | |
|---|---|
| C-Quelle (10 g/l) | Wachstum * |
| Kontrolle (keine C-Quelle) | - |
| D-Glucose | + |
| L-Arabinose | + |
| L-Rhamnose | + |
| D-Fructose | + |
| L-Galactose | + |
| Raffinose | + |
| D-Mannit | + |
| meso-Inosit | + |
| Salicin | + |
| Saccharose | + |
| Ribose | + |
| Mannose | + |
| Maltose | + |
| Mellibiose | + |
| Cellulose | - |
| Acetat | - |

)* + = Wachstum, - = kein Wachstum.

3. Besonders geeignetes Medium für Wachstum und Sporulation

ISP 3 (Hafermehl-Agar)

20 g Hafermehl werden in 1000 ml $H_2O$ deion. suspendiert und 20 Minuten gekocht. Anschließend wird filtriert, 1 ml Spurenelementlösung für ISP 3 und 18 g Agar zugegeben, und der pH-Wert auf 7,2 eingestellt, und bei 121°C 15-20 Minuten autoklaviert.

Spurenelementlösung für ISP 3

| | |
|---|---|
| $FeSO_4$ . 7 $H_2O$ | 0,1 g |
| $MnCl_2$ . 4 $H_2O$ | 0,1 g |
| $ZnSO_4$ . 7 $H_2O$ | 0,1 g |
| $H_2O$ deion. | 100 ml |

Weitere Medien s. Int. J. Syst. Bact. 16, 313-340 (1966).

Der Stamm BS 1261, isoliert aus einer Erdprobe aus Neuseeland, laßt sich aufgrund der morphologischen Daten in die graue Serie der Streptomyceten (Cinereus-Gruppe) einordnen.

Taxonomische Bezeichnung: Strentomyces sp.

Efomycin G wird erfindungsgemäß durch die Fermentation geeigneter Mikroorganismen, wie dem Streptomycetenstamm BS 1261 oder dessen Mutanten oder Varianten erzeugt.

Das erfindungsgemäße Fermentationsverfahren kann mit Hilfe fester, halbfester oder flüssiger Nährmedien durchgeführt werden. Bevorzugt werden wäßrig-flüssige Nährmedien verwendet.

Die Beimpfung der Nährmedien erfolgt nach allgemein üblichen Methoden, z.B. über Schrägröhrchen oder Kolbenkulturen.

Die Kultur erfolgt unter aeroben Bedingungen und kann gemäß den allgemein üblichen Methoden wie unter Verwendung von Schüttelkulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Fermentation kontinuierlich oder diskontinuierlich durchzuführen. Vorzugsweise wird diskontinuierlich gearbeitet.

Die Kultur wird in Nährmedien durchgeführt, welche bekannterweise zur Kultivierung von Mikroorganismen der Ordnung Actinomycetales verwendet werden. Das Nährmedium muß eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen infrage. Beispielsweise seien Kohlenhydrate, insbesondere Polysaccharide, wie Stärke oder Dextrine, Disaccharide, wie Maltose oder Lactose, Monosaccharide, wie Glucose oder Xylose, Alkohole, wie Mannit oder Glycerin sowie natürlich vorkommende Gemische, wie Malzextrakt, Melasse oder Molkepulver genannt. Als Stickstoffquellen kommen alle üblichen organischen und anorganischen Stickstoffquellen infrage. Beispielsweise seien Eiweißstoffe, Eiweißhydrolysate, Aminosäuren, Nucleosidbasen, wie Cytosin oder Uracil sowie Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt, stickstoffhaltige Salze wie z.B. Ammoniumsalze und Nitrate, aufgeführt. Die Mineralsalze, welche im Nährmedium enthalten sein sollten, liefern z.B. folgende Ionen:

$Mg^{++}$, $Na^+$, $K^+$, $Ca^{++}$, $NH_4^+$, $Cl^-$, $SO_4^{--}$, $PO_4^{---}$ und $NO_3^-$

sowie Ionen der üblichen Spurenelemente, wie Cu, Fe, Mn, Mo, Zn, CO, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Bei der Durchführung des Verfahrens kann es günstig sein, zu Beginn der Kultivierung nur relativ geringe Konzentrationen der löslichen Nährlösungsbestandteile zu verwenden und dann im Laufe der ersten 3 Kultivierungstage durch häufigere Zusätze diese Bestandteile in Form steriler, relativ konzentrierter Lösungen dem Kulturansatz fraktioniert zuzufüttern.

Der pH-Wert der wachsenden Kulturen sollte vorzugsweise zwischen etwa 5 und etwa 10, insbesondere zwischen 6,5 und 8,0 gehalten werden. Ein zu starker pH-Abfall in den sauren Bereichen kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säuren, z.B. $H_2SO_4$, oder sterile Laugen, z.B. NaOH in Abständen in die Kulturlösung eingespritzt werden.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 24°C und etwa 34°C, vorzugsweise zwischen 26°C und 32°C liegen, besonders bevorzugt liegt sie bei etwa 28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindungen im allgemeinen ihr Maximum etwa 1 bis 10, vorzugsweise etwa 4 bis 7 Tage nach Züchtungsbeginn erreicht. Das gewünschte Endprodukt der Fermentation kann mit Hilfe von dünnschichtchromatographischen und hochdruckflüssigkeitschromatographischen Untersuchungen oder biologischen Testverfahren bestimmt werden.

Wie allgemein bei mikrobiologischen Verfahren üblich, sollten Fremdinfektionen der Kulturmedien vermieden werden. Hierzu werden die üblichen Vorkehrungen getroffen, wie Sterilisation der Nährmedien, Kulturgefäße sowie der für die Belüftung notwendigen Luft. Zur Sterilisation der Vorrichtungen können z.B.

die Dampfals auch die Trockensterilisation verwendet werden, wobei die Temperaturen vorzugsweise bei 100 bis 140°C, insbesondere bei 120 bis 130°C liegen können.

Falls bei der Kultivierung in unerwünschter Menge Schaum entsteht, können die üblichen chemischen Schaumdämpfungsmittel, z,B. flüssige Fette und Öle, wie Öl-Wasser-Emulsionen, Paraffine, höhere Alkohole, wie Octadecanol, Siliconöle, Polyoxyethylen- bzw. Polyoxypropylenverbindungen (z.B. in Mengen bis etwa 1 %) zugesetzt werden. Schaum kann auch mit Hilfe der üblichen mechanischen Vorrichtungen (welche z.B. Zentrifugalkräfte benutzen) gedämpft oder beseitigt werden.

Die erfindungsgemäße Verbindung kann aus dem Kulturmedium mit Hilfe von Chromatographieverfahren erfolgen. Bei allen Isolierungs- und Reinigungsoperationen ist darauf zu achten, daß die pH-Werte im neutralen Bereich liegen. Vorzugsweise werden pH-Werte zwischen 7 und 8 eingehalten. Zur Einstellung des pH-Wertes können anorganische und organische Basen verwendet werden, wie Alkalibasen z.B. NaOH, KOH oder organische Amine, wie Triethylamin; anorganische Säuren wie z.B. HCl und organische Säuren wie z.B. Essigsäure.

Um bei den oben angegebenen Isolierungs- und Reinigungsmethoden die Fraktionen herauszufinden, in welchen die erfindungsgemäße Verbindung in höchster Konzentration bzw. Reinheit vorliegen, können die üblichen physikalisch chemischen Methoden z.B. Messen einer charakeristischen Bande im Spektrum oder der $R_f$-Werte, Bestimmung der antibakteriellen Aktivität usw. herangezogen werden. Diese Methoden können auch verwendet werden, um in Routineverfahren für die Produktion von Efomycin G geeignete Mikroorganismen aufzufinden.

Die Isolierung und Reinigung der erfindungsgemäßen Verbindung kann z.B. im Falle, daß ein flüssiges wäßriges Nährmedium verwendet wird, wie folgt vorgenommen werden:
Da Efomycin G sowohl im Kulturüberstand als auch im Myzel zu finden ist, kann es mit Hilfe von üblichen Extraktionsverfahren, Fällungsverfahren und/oder Chromatographieverfahren aus dem Fermentationsansatz isoliert und gegebenenfalls gereinigt werden. Die Chromatographie kann in Form der Säulenchromatographie durchgeführt werden.

Es werden zur Isolierung der erfindungsgemäßen Verbindungen Chromatographie-Verfahren verwendet, und zwar unspezifische Adsorption an Sorbentien wie Kieselgel.

In einer bevorzugten Ausführungsform wird das Mycelium aus der Kulturbrühe, vorzugsweise durch Zentrifugation abgetrennt und mit einem, mit Wasser mischbaren Lösungsmittel mehrfach, vorzugsweise zweimal extrahiert. Als Lösungsmittel können ($c_1$-$C_4$)-Alkylalkohole, $C_{1-4}$-Ketone, besonders bevorzugt Aceton verwendet werden. Die wäßrig-organische Lösung wird im Vakuum, z.B. etwa auf 1/20 des Volumens der Kulturbrühe konzentriert und gefriergetrocknet.

Dieses Rohpräparat wird in Wasser suspendiert und die Efomycin G mit einem mit Wasser nicht mischbaren Lösungsmittel, wie z.B. Chlorkohlenwasserstoffe wie Dichlormethan extrahiert. Aus diesem Extrakt kann Efomycin G wie in den Beispielen beschrieben, durch Chromatographie an Kieselgel isoliert werden.

Die erfindungsgemäße Verbindung zeigt eine gute antibakterielle Wirkung vor allem gegen grampositive Keime. Besonders erwähnt werden soll ihre Eignung zur Verhinderung und Heilung der Schweinedysenterie und von Ketose.

Der Wirkstoff wird als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Der Wirkstoff wird bei Nutztieren, bevorzugt bei Wiederkäuern wie z.B. Rindern, Schafen und Ziegen verwendet.

Der Wirkstoff wird unabhängig vom Geschlecht der Tiere während allen Leistungsphasen der Tiere eingesetzt. Bevorzugt wird der Wirkstoff während der intensiven Leistungsphase eingesetzt. Die intensive Wachstums-und Leistungsphase dauert je nach Tierart von einem Monat bis zu 10 Jahren.

Die Menge des Wirkstoffes, die den Tieren zur Erreichung des gewünschten Effektes verabreicht wird, kann wegen der günstigen Eigenschaften des Wirkstoffes weitgehend variiert werden. Sie liegt vorzugsweise bei etwa 0,001 bis 500 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Leistungsphase, dem Gesundheitszustand und der Art der Haltung und Fütterung der Tiere ab und sind durch jeden Fachmann leicht zu ermitteln.

Der Wirkstoff wird den Tieren nach den üblichen Methoden verabreicht. Die Art der Verabreichung hängt insbesondere von der Art, dem Verhalten und dem Gesundheitszustand der Tiere ab.

Der Wirkstoff kann einmalig verabreicht werden. Der Wirkstoff kann aber auch während der ganzen oder während eines Teils der Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

6

Die Verabreichung erfolgt oral in dafür geeigneten Formulierungen oder in reiner Form.

Der Wirkstoff kann in den Formulierungen allein oder in Mischung mit anderen leistungsfördernden Wirkstoffen, mineralischen Futtermitteln, Spurenelement-Verbindungen,Vitaminen, Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind:

z.B. Antibiotika wie Tylosin, Virginiamycin und Monensin, Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid. Vitamine sind z.B. Vitamin A, Vitamin $D_3$, Vitamin E, B-Vitamine, Vitamin C.

Nicht-Protein-Verbindungen sind z.B. Biuret, Harnstoff. Farbstoffe sind z.B. Carotinoide wie Citranaxanthin, Zeaxanthin, Capsanthin.

Antioxidantien sind z.B. Aethoxyquin, Butylhydroxy-Toluol.

Aromastoffe sind z.B. Vanillin.

Emulgatoren sind z.B. Ester der Milchsäure, Lecithin.

Fließhilfsstoffe sind z.B. Natriumstearat, Calciumstearat.

Konservierungsstoffe sind z.B. Zitronensäure, Propionsäure.

Preßhilfsstoffe sind z.B. Ligninsulfonate, Celluloseether.

Die Verabreichung des Wirkstoffes kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäure z.B. DL-Methionin, Salze wie Kalk und Kochsalz, Zum Futter zählen auch Ergänzungs-, Fertig- und Mischfuttermittel. Diese enthalten Einzelfuttermittel in einer Zusammensetzung die eine ausgewogene Ernährung hinsichtlich der Energie- und Proteinversorgung sowie der Versorgung mit Vitaminen, Mineralsalzen und Spurenelementen sicherstellen.

Die Konzentration des Wirkstoffs im Futter beträgt normalerweise etwa 0,01-500 ppm, bevorzugt 0,1-50 ppm.

Der Wirkstoff kann als solcher oder in Form von Prämixen oder Futterkonzentraten dem Futter zugesetzt werden.

Beispiel für die Zusammensetzung eines Futters für Rinder, das den erfindungsgemäßen Wirkstoff enthält:

69,95 % Futtergetreideschrot, 10 % gemahlene Maiskolben, 8 % Soyabohnenmehl, 5 % Luzernemehl, 5 % Melasse, 0,6 % Harnstoff, 0,5 % Calciumphosphat, 0,5 % Calciumcarbonat, 0,3 % Kochsalz, und 0,15 % Prämix. Das Prämix enthält 70.000 i.E. Vitamin A, 7000 i.E. Vitamin D3, 100 mg Vitamin E, 50 mg Mangan, 30 mg Zink und 0,06 mg Kobalt. Der Wirkstoff wird dem Prämix in erforderlicher Menge beigemischt.

Die Herstellung und biologische Wirkung der neuen erfindungsgemäßen Verbindung kann durch die folgenden Beispiele erläutert werden:

Beispiel 1

Herstellung des Inoculums

Zellen von Streptomyces sp. BS 1261 (DSM 3200) wurden aus einem Schrägröhrchen in 1000 ml Erlenmeyer-Kolben übertragen, die je 150 ml folgender steriler Nährlösung enthielten:
CASO® der Firma Merck, Darmstadt der Zusammensetzung:

| | |
|---|---|
| Pepton aus Casein | 15 g |
| Pepton aus Sojamehl | 5 g |
| Glucose | 2,5 g |
| NaCl | 5 g |
| Wasser | ad 1000 ml |

Die Kolben wurden 3 Tage bei 28°C mit 250 Upm auf der rotierenden Schüttelmaschine inkubiert. 2 Kolben der resultierenden Kultur wurden vereinigt und dienten als Inoculum für einen 30 l Fermenter, der 20 l obiger, steriler Nährlösung, versetzt mit 20 ml SAG 5693 (Union Carbide), enthielt.

Die Fermentation wurde bei 28°C mit einer Belüftungsrate von 10 l/min (0.5 vvm) Luft und einem Überlagerungsdruck von 0,5 bar durchgeführt. Die Drehzahl des Blattrührers betrug 300 Upm. Nach 48 Stunden diente die so gewonnene Kultur als Inoculum für die Tankfermentation.

Beispiel 2

Tankfermentation

Mit 20 Litern des nach Beispiel 1 hergestellten Inoculums wurde ein 300 l Kessel beimpft, der 200 l steriles Nährmedium folgender Zusammensetzung enthielt:

| Magermilchpulver | 10 g |
|---|---|
| Hefeautolysat | 1,5 g |
| Dextrin | 40 g |
| D-Glucose | 5 g |
| SAG 5693 (Union Carbide) | 1 ml |
| Wasser | ad 1000 ml |

Der pH-Wert des Mediums lag nach Sterilisation bei 6.6. Die Fermentation wurde bei 28°C mit einer Drehzahl des Blattrührers von 100 Upm, einer Belüftungsrate von 100 l/min (0.5 vvm) Luft und einem Überlagerunsdruck von 1.0 bar durchgeführt, bis nach 3 - 5 Tagen Efomycin G nachgewiesen werden konnte.

Beispiel 3

Die gemäß Beispiel 2 erhaltene Kulturbrühe einer 200-Liter Fermentation wird bei pH 7-7.5 mit 200-250 l/h in einem Westfalia-Separator separiert. Das Mycel wird mit dem doppelten Volumen Aceton 30 Minuten bei Raumtemperatur gerührt und zentrifugiert. Der Rückstand wird erneut mit dem doppelten Volumen Aceton gerührt und zentrifugiert. Die vereinigten Zentrifugate werden mit reduziertem Druck bei 40°C Badtemperatur konzentriert. Das Konzentrat wird mit 10 Liter Wasser versetzt und dreimal mit je 10 Liter Dichlormethan extrahiert. Die vereinigten organischen Phasen trocknete man über Natriumsulfat, filtrierte und engte unter reduziertem Druck bei max. 40° Badtemperatur auf 3 Liter ein. Das Konzentrat ließ man in ca. 30 Liter Leichtbenzin unter Rühren einfließen. Der Niederschlag wurde abfiltriert und im Vakuum bei 40°C getrocknet. Ausbeute: 61 g gelbliches Pulver.

Beispiel 4

Efomycin G isolierte man aus dem nach Beispiel 3 gewonnenen Rohprodukt durch präparierte Flüssigchromatografie.

Parameter:

| | |
|---|---|
| Fließmittel | A: 5 mM Citronensäure: Acetonitril = 6 : 4 |
| | B: 5 mM Citronensäure: Acetonitril = 4 : 6 |
| Gradient: | nach 3 Minuten isokratischem Lauf mit Fließmittel A:B = 4:1 linearer Gradient in 1 von A:B = 4:1 auf A:B = 1:9 |
| Fließrate: | 20 ml/min |
| Detektion: | UV 254 nm |
| Säule: | 21.6 x 250 mm |
| stationäre Phase: | Zorbax® C8 8 $\mu$m (DuPont) |

Pro Trennung wurden ca. 30 mg des nach Beispiel 3 gewonnenen Rohproduktes gelöst in je 2 ml Tetrahydrofuran/Wasser = 2/1 eingesetzt. Das Säulenelat wurde fraktioniert aufgefangen und einer analytischen Hochdruckflüssigchromatographie gemäß folgender Parameter unterworfen.

| | |
|---|---|
| Fließmittel A: | 5 m M Citronensäure |
| | 0,1 M $NaClO_4$ |
| Fließmittel B: | Acetonitril |
| Gradient: | linear 52-75 % B in 18 Minuten |

8

Fließrate:                1,5 ml/Min.
Detektion:             UV 254 nm
Stationäre Phase:    Nucleosil® 10C18
Säule:                 4,6 x 250 ml

Fraktionen, die reines Efomycin G enthielten, wurden vereinigt und unter reduziertem Druck bei 40°C Badtemperatur auf 1/3 des Anfangsvolumens eingeengt. Das als farbloser Niederschlag anfallende Efomycin G wurde abfiltriert und im Hochvakuum bei 40°C getrocknet. Ausbeute: 5 mg Efomycin G; Reinheit nach HPLC besser als 85 %.

Beispiel A:

Einem Hammel, der pro Tag 650 g grob gemahlenes Schaf-Fertigfutter und 250 g Trockengrün Cobs erhielt, wurde Pansenflüssigkeit durch eine Pansenfistel entnommen. Das Fertigfutter wurde über einen Futterautomaten in 12 gleichen Portionen in zweistündlichem Abstand und die Cobs in 2 gleichen Portionen um 8.30 und 16.15 Uhr verabreicht. Die Pansenflüssigkeit wurde unmittelbar nach der Gewinnung folgender Behandlung unterworfen: 2,5 ml des Panseninokulums wurden in einem mit Kohlendioxid begasten Reagenzröhrchen von 13 ml Volumen vorgelegt, das darüber hinaus folgende Zusätze enthielt:
100 mg fein gemahlenes Schaf-Fertigfutter
7,5 ml Pufferlösung
0,5 ml 5 %iges wäßriges Ethanol mit bzw. ohne Efomycin G.
Die Zusammensetzung der Pufferlösung, welche vor Anfang des Versuches mit Kohlendioxid gesättigt wurde, war wie folgt:

$$Na_2HPO_4 \qquad 4,61 \; g \; per \; Liter \; Wasser$$
$$NaHCO_3 \qquad 12,25 \; g \; " \qquad " \qquad "$$
$$NaCl \qquad 0,59 \; g \; " \qquad " \qquad "$$
$$KCl \qquad 0,71 \; g \; " \qquad " \qquad "$$
$$MgCl_2 \qquad 0,32 \; g \; " \qquad " \qquad "$$
$$CaCl_2 \qquad 0,13 \; g \; " \qquad " \qquad "$$

Jedes Reagenzröhrchen wurde mit einem Bunsen-Stopfen verschlossen und bei 39°C bebrütet. Nach 2, 4, 6 und 8 Stunden wurden die Ansätze von Hand geschüttelt. Nach 24-stündiger Bebrütung wurde 1,0 ml der Fermentationsflüssigkeit aus den Ansätzen entnommen und in ein Eppendorf-Gefäß pipettiert, das 0,2 ml 10 %ige Phosphorsäure (enthaltend 5,7 mcmol 2-Methylveriansäure) enthielt. Die Proben wurden bei 11 000 g zentrifugiert und aus dem Überstand die flüchtigen Fettsäurekonzentrationen gaschromatographisch bestimmt.

Es wurde bei jedem Versuch das Verhältnis Essigsäure zu Propionsäure bestimmt. Der bei den Negativkontrollen erhaltene Wert wurde mit 100 angesetzt und die Abweichungen im Verhältnis dazu angegeben. Je mehr Propionsäure gebildet wurde, um so niedriger liegt das Verhältnis Essigsäure zu Propionsäure und um so kleiner wird die Verhältniszahl im Vergleich zur Kontrolle (niedrige Verhältniszahl = verringertes Essigsäure/Propionsäure-Verhältnis = verbesserte Futterverwertung).

Zusätzlich werden bei jedem Versuch die Konzentrationen der Gesamtfettsäuren im Vergleich zur Kontrolle (= 100) angegeben.

Tabelle

| Menge (µg/Ansatz) | Essigsäure-Propionsäure-Verhältnis | Gesamtfettsäuren |
|---|---|---|
| Kontrolle | 100 | 100 |
| 250 | 64,3 | 101,5 |
| 500 | 58,8 | 103,8 |

**Patentansprüche**

1. Efomycin G, das durch die folgenden chemischen und physikalischen Eigenschaften charakterisiert ist:
   1. Die Summenformel: $C_{53}H_{86}O_{18}$
   2. Das Massenspektrum (Fast Atom Bombardment) Molmasse + $Na^+$: 1033
   3. Das $^1$H-Kernresonanzspektrum, angegeben in Teilen pro Million, gemäß Fig. 1.
   Es wurde in einem AM 300 der Fa. Bruker bei MHz an einer Lösung von Efomycin G in deuteriertem Chloroform mit Tetramethylsilan als innerem Standard aufgenommen.
   4. Das $^{13}$C-Kernresonanzspektrum, angegeben in Teilen pro Million, gemäß Fig. 2.
   Es wurde in einem AM 300 der Fa. Bruker bei 75,48 MHz an einer Lösung von Efomycin G in deuteriertem Chloroform und deuteriertem Methanol mit Tetramethylsilan als innerem Standard aufgenommen.
   Die chemischen Verschiebungen der 13C-NMR Signale von Efomycin G gemäß Fig. 2 betragen im einzelnen:

   | | | | | |
   |---|---|---|---|---|
   | 170.0 | 77.9 | 65.9 | 32.9 | 7.0 |
   | 145.4 | 73.4 | 48.5 | 19.4 | |
   | 144.7 | 71.2 | 43.6 | 19.1 | |
   | 131.9 | 70.6 | 41.9 | 16.8 | |
   | 121.1 | 70.1 | 41.1 | 16.7 | |
   | 99.6 | 69.9 | 38.8 | 15.1 | |
   | 99.5 | 66.9 | 38.5 | 13.4 | |
   | 93.6 | 66.5 | 36.2 | 9.0 | |
   | 93.3 | 66.4 | 33.0 | 8.9 | |

   (angegeben sind ppm-Werte relativ zu Tetramethylsilan bei 0 ppm)
   5. Das UV-Absorptionsmaximum bei 251 - 254 nm in methanolischer Lösung.

2. Verfahren zur Herstellung von Efomycin G gemäß Anspruch 1, dadurch gekennzeichnet, daß man geeignete Mikroorganismen der Familie der Streptomycetaceae in einem Nährmedium welches assimilierbare Kohlenstoff- und Stickstoffquellen sowie Mineralsalze enthält unter aeroben Bedingungen kultiviert, das entstandene Gemisch der Efomycine in Tetrahydrofuran/Wasser 2:1 aufnimmt, und das Efomycin G mit einer Flüssigkeitschromatographie auf Zorbax$^R$ mit einem Citronensäure/Acetonitril Gradienten eluiert.

3. Verfahren gemäß Anspruch 2, dadurch gekennzeichnet, daß es mit dem Stamm BS 1261 (entsprechend DSM Nr. 3200) durchgeführt wird.

**Claims**

1. Efomycin G, which is characterized by the following chemical and physical properties:
   1. The empirical formula: $C_{53}H_{86}O_{18}$
   2. The mass spectrum (fast atom bombardment) Molecular weight + $Na^+$: 1033
   3. The $^1$H-nuclear magnetic resonance spectrum, stated in parts per million, according to Figure 1.
   This was recorded in an AM 300 from Bruker at MHz on a solution of efomycin G in deuterated chloroform with tetramethylsilane as the internal standard.
   4. The $^{13}$C-nuclear magnetic resonance spectrum, stated in parts per million, according to Figure 2.
   This was recorded in an AM 300 from Bruker at 75.48 MHz on a solution of efomycin G in deuterated chloroform and deuterated methanol with tetramethylsilane as the internal standard.
   The chemical shifts of the $^{13}$C-NMR signals of efomycin G according to Figure 2 are, in detail:

| 170.0 | 77.9 | 65.9 | 32.9 | 7.0 |
|-------|------|------|------|-----|
| 145.4 | 73.4 | 48.5 | 19.4 | |
| 144.7 | 71.2 | 43.6 | 19.1 | |
| 131.9 | 70.6 | 41.9 | 16.8 | |
| 121.1 | 70.1 | 41.1 | 16.7 | |
| 99.6 | 69.9 | 38.8 | 15.1 | |
| 99.5 | 66.9 | 38.5 | 13.4 | |
| 93.6 | 66.5 | 36.2 | 9.0 | |
| 93.3 | 66.4 | 33.0 | 8.9 | |

(ppm values relative to tetramethylsilane at 0 ppm are stated)

5. The UV absorption maximum at 251 - 254 nm in methanolic solution.

**2.** Process for the preparation of efomycin G according to Claim 1, characterized in that suitable microorganisms of the Streptomycetaceae family are cultured under aerobic conditions in a nutrient medium containing assimilable carbon and nitrogen sources and mineral salts, the resulting mixture of the efomycins is taken up in tetrahydrofuran/water 2:1, and the efomycin G is eluted with a liquid chromatography on Zorbax® with a citric acid/acetonitrile gradient.

**3.** Process according to Claim 2, characterized in that it is carried out with the strain BS 1261 (corresponding to DSM No. 3200).

**Revendications**

**1.** Efomycine G, qui est caractérisée par les propriétés chimiques et physiques suivantes :

1. formule brute : $C_{53}H_{86}O_{18}$.

2. Spectre de masse (Fast Atom Bombardment) masse molaire + $Na^+$ : 1033.

3. Spectre de résonance magnétique des protons, indiqué en parties par millions, conforme à la figure 1.

Il a été enregistré dans un appareil AM 300 de la firme Bruker à 75,48 MHz sur une solution d'éfomycine G dans du deutérochloroforme avec le tétraméthylsilane comme substance interne de référence.

4. Spectre de résonance magnétique des noyaux de $^{13}C$, indiqué en parties par million, conforme à la figure 2.

Il a été enregistré dans un appareil AM 300 de la firme Bruker à 75,48 MHz sur une solution d'éfomycine G dans du deutérochloroforme et du deutérométhanol avec du tétraméthylsilane comme substance interne de référence.

Les déplacements chimiques des signaux de $^{13}C$-RMN de l'éfomycine G selon la figure 2 ont en particulier les valeurs :

| 170,0 | 77,9 | 65,9 | 32,9 | 7,0 |
|-------|------|------|------|-----|
| 145,4 | 73,4 | 48,5 | 19,4 | |
| 144,7 | 71,2 | 43,6 | 19,1 | |
| 131,9 | 70,6 | 41,9 | 16,8 | |
| 121,1 | 70,1 | 41,1 | 16,7 | |
| 99,6 | 69,9 | 38,8 | 15,1 | |
| 99,5 | 66,9 | 38,5 | 13,4 | |
| 93,6 | 66,5 | 36,2 | 9,0 | |
| 93,3 | 66,4 | 33,0 | 8,9 | |

(ces valeurs sont indiquées en ppm par rapport au tétraméthylsilane pour 0 ppm).

5. Maximum d'absorption UV à 251 - 254 nm en solution méthanolique.

**2.** Procédé de préparation d'éfomycine G suivant la revendication 1, caractérisé en ce qu'on cultive des micro-organismes appropriés de la famille des Streptomycetaceae dans un milieu nutritif qui contient des sources assimilables de carbone et d'azote ainsi que des sels minéraux dans des conditions aérobies, on reprend le mélange d'éfomycines produit dans un mélange à 2:1 de tétrahydrofuranne et d'eau et on élue l'éfomycine G par chromatographie en phase liquide sur Zorbax$^R$ selon un gradient d'acide citrique/acétonitrile.

3. Procédé suivant la revendication 2, caractérisé en ce qu'il est mis en oeuvre avec la souche BS 1261 (correspondant au numéro DSM 3200).

FIG. 1

FIG. 2